(19) Europäisches Patentamt
European Patent Office
Office européen des brevets

(11) Publication number: 0 144 084 A2

# (12) EUROPEAN PATENT APPLICATION

(21) Application number: 84114528.7

(22) Date of filing: 30.11.84

(51) Int. Cl.[4]: G 01 N 33/531
G 01 N 33/549

(30) Priority: 30.11.83 JP 224509/83

(43) Date of publication of application: 12.06.85 Bulletin 85/24

(84) Designated Contracting States: DE FR GB IT

(71) Applicant: Kabushiki Kaisha Toshiba
72, Horikawa-cho Saiwai-ku
Kawasaki-shi Kanagawa-ken 210(JP)

(72) Inventor: Ishimori, Yoshio
c/o Kabushiki Kaisha Toshiba 72, Horikawa-cho
Saiwai-ku Kawasaki-shi Kanagawa-ken(JP)

(72) Inventor: Notsuki, Masato
c/o Kabushiki Kaisha Toshiba 72, Horikawa-cho
Saiwai-ku Kawasaki-shi Kanagawa-ken(JP)

(72) Inventor: Koyama, Masao
c/o Kabushiki Kaisha Toshiba 72, Horikawa-cho
Saiwai-ku Kawasaki-shi Kanagawa-ken(JP)

(72) Inventor: Yasuda, Tatsuji
c/o Kabushiki Kaisha Toshiba 72, Horikawa-cho
Saiwai-ku Kawasaki-shi Kanagawa-ken(JP)

(74) Representative: Hansen, Bernd, Dr.rer.nat. et al,
Hoffmann, Eitle & Partner Patentanwälte Arabellastrasse 4
D-8000 München 81(DE)

## (54) Reagent for immunoassay and analytical method using the same.

(57) A reagent for an immunoassay comprises liposomes; a hydrophilic antigen, antibody or at least a part of the antibody immobilized on the liposomes by a cross-linking method or an active lipid method; and a hydrophilic marker material enclosed in the liposomes, and a method for an immunoassay comprises the steps of:

mixing the above reagent with a sample including an antigen or antibody and a complement; and

measuring the marker material released out of the liposomes in order to quantitatively determine the antigen or antibody in the sample.

The reagent of this invention could allow an accurate determination of the antigen or antibody in a homogeneous system with a high detection sensitivity in a short time.

EP 0 144 084 A2

Reagent for immunoassay and analytical method using the same

BACKGROUND OF THE INVENTION

This invention relates to a reagent for immunoassay and an analytical method using the same, more particularly to a reagent for an immunoassay which is utilized to quantitatively analyze a specific antigen or antibody, and an analytical method in which the reagent is employed.

In recent years, a variety of tumor markers have been found out, as researches on cancers make advances. Typical examples of such tumor markers include α-fetoprotein (AFP), carcinoembryonic antigen (CEA), basic fetoprotein (BFP) and pancreatic oncofetal antigen (POA). Concentrations of these tumor markers in normal human sera are very low (e.g., the concentration of AFP is 10 ng/ml or less). On the other hand, these concentrations of tumor patients are about 10 times as high as the normal human sera in most cases. At any rate, with regard to the quantitative analysis of the tumor markers, a very high detection sensitivity is required.

In order to satisfy this requirement, there has been

developed radio-immunoassay (RIA) in which an antigen or antibody labelled by a radioactive material is employed. However, the RIA method has been troublesome in points of handling and disposal treatment. For this reason, there has been suggested another immunoassay in which an antigen or antibody labelled with a variety of materials such as enzymes and fluorescent materials in place of the radioactive materials. However, this immunoassay technique also has the drawback that a free antibody and a combined antibody must be separated from each other in any manner. Further, an EMIT method, which has been disclosed in Rosenthal A. F., Vargas M. G. and Klass C. S. (1976), Clin. Chem., Vol. 22, p. 1899, is an epochal method by which measurement can be made in a uniform system without requiring any separation process, but it cannot be fundamentally applied to a protein antigen or antibody having a high molecular weight.

Haxby J. A., Kinsky C. B. and Kinsky S. C. (1968), Biochemistry, Vol. 61, p. 300 has revealed a method by which there is prepared liposomes where a fat-soluble antigen is taken in its membrane and glucose is contained, and glucose discharged from the liposomes due to its breakage caused by an antigen-antibody reaction is quantitatively determined in order to accomplish the quantitative analysis of an antibody. However, if the measurement of the tumor marker is attempted, it will be necessary to support the marker itself or the antibody against this marker, i.e. an immunogloblin which is a kind of protein, on the liposomes. Up to now, the liposomes incorporated into hydrophobic proteins have been feasible, but it has not been reported yet that water-soluble protein-bearing liposomes are contemplated for the aim of immunoassays of antigens or antibodies. This reason is that a technique of supporting the water-soluble protein on liposomes has not been established.

Further, in Japanese Provisional Patent Publication (KOKAI) No. 132564/1981 entitled "Product for immunological analysis and method for preparing the same", there is disclosed a method by which liposomes supporting an antigen or antibody thereon and including an enzyme therein are used to carry out an immunoassay. In this suggested method, a protein is supported on the liposomes by using a bifunctional cross-linking agent such as glutaraldehyde. However, it has been realized from the present inventors' researches that if the antigen is supported on the liposomes with the aid of the cross-linking agent, the activity of the antigen will be generally reduced and the liposomes breakage to be caused by the antigen-antibody reaction will not be achieved.

Furthermore, conventional immunoassay techniques have drawbacks of usually taking a long time and not being able to measure automatically a large amount of samples.

## SUMMARY OF THE INVENTION

An object of this invention is to provide a method for an immunoassay of a homogeneous system by which an antigen or antibody in a sample can be quantitatively measured in a short time without any separating operations by using a developed reagent for the immunoassay in which a hydrophilic antigen or antibody is supported on liposomes.

The present inventors have intensively conducted researches to achieve the above-mentioned object, and as a result, they have now succeeded in immobilizing the antibody, at least a part of the antibody or antigen corresponding to the antigen or antibody in a sample on liposomes which will be dissolved by a function of complement, without lowering the activity of the antibody or antigen, and have found out that the object of this invention is achieved by enclosing a marker material in

the liposomes. The completion of this invention has just been accomplished on the basis of these research results.

That is to say, the reagent used with complement to analyse an antigen or antibody in a sample for immunoassay of this invention comprises liposomes; a hydrophilic antigen, antibody or at least a part of the antibody immobilized on the liposomes by a cross-linking method or an active lipid method; and a hydrophilic marker material enclosed in the liposomes.

Further, this immunoassay comprises by mixing, with a sample including an antigen or antibody and a complement, a reagent for the immunoassay comprising liposomes, a hydrophilic antigen, antibody or at least a part of the antibody immobilized on the liposomes by a cross-linking method or an active lipid method, and a hydrophilic marker material enclosed in the liposomes; and measuring the marker material released from the liposomes in order to quantitatively determine the antigen or antibody concentration in the sample.

BRIEF DESCRIPTION OF THE DRAWINGS

Figure 1 is a diagram showing the correlation between a dilute magnification of an anti-human IgG antibody and a relative fluorescent intensity in the case that the anti-human IgG antibody was determined using IgG immobilized liposomes;

Figure 2 is a diagram showing the correlation between a dilute magnification of a serum and a relative fluorescent intensity in the case that the human IgG in the serum was determined using human IgG immobilized liposomes;

Figure 3 is a characteristic diagram showing the correlation between a concentration of an anti-human IgG antibody and a relative absorbance in the case that the anti-human IgG antibody was determined using human IgG immobilized liposomes;

Figure 4 is a characteristic diagram showing the correlation between a concentration of an anti-human IgG antibody and a relative release rate of a marker material in the case that the anti-human IgG antibody was determined using human IgG immobilized liposomes, where the curve a shows the case that SPDP was used as a cross-linking agent and curve b shows the case that GA was used;

Figure 5 is a characteristic diagram showing the correlation between a concentration of immobilized human IgG on a double membrane of the liposomes and a relative release rate of the marker material;

Figure 6 is a characteristic diagram showing the correlation between a concentration of an immobilized human IgG antigen and a relative release rate of the marker material;

Figure 7 is a characteristic diagram showing the correlation between a complement value and a relative release rate of the marker material;

Figure 8 is a characteristic diagram showing the correlation between a concentration of the human IgG antigen and a relative release rate of the marker material in the case that human IgG was measured using the anti-human IgG bearing-liposomes;

Figure 9 is a characteristic diagram showing the correlation between a concentration of an anti-human IgG antibody and a relative release rate of a marker material in the case that the anti-human IgG antibody was determined

using the human IgG bearing-liposomes which was enclosed glucose oxydase as the marker material;

Figure 10 is a characteristic diagram showing the correlation between a concentration of an anti-human IgG antibody and a relative release rate of a marker material in the case that the anti-human IgG antibody was determined using the human IgG bearing-liposomes which was enclosed luciferin as the marker material;

DESCRIPTION OF THE PREFERRED EMBODIMENTS

Liposomes for an immunoassay of this invention have a wide meaning including even a red blood cell ghost membrance. As such liposomes, any ones are acceptable so long as it has been conventionally used, but the liposomes composed of a phospholipid or glycolipid and cholesterol are more preferable from the viewpoint of the stability of the reagent. For example, when synthesized from the phospholipid and cholesterol, the particularly stable liposomes can be prepared on the condition that the molar ratio of phospholipid : cholesterol is between 1 : 0.1 and 1 : 5.0, more preferably 1 : 1 or so. As for a fatty acid residue in the phospholipid, the number of its carbon atoms is preferably 12 to 18, and it is more preferable that this number is even. Further, in cases that glycolipid is substituted for phospholipid or both of phospholipid and glycolipid are used, the molar ratio of these lipids to cholesterol is preferable in the range as mentioned above.

Antigens, antibodies or at least a part of the antibody to be immobilized on the liposomes include antigens for tumor markers (the above-mentioned AFP, BEP, CEA, POA, etc.), immunoglobulins (IgA, IgE, IgG, IgM, etc.), hormones (insulin, $T_3$, $T_4$, etc.), drugs (phenitoin, digoxin, etc.) and the like; or antibodies against the

aforesaid antigens. However, these antigens, antibodies or at least a part of the antibodies are required to be hydrophilic.

In the reagent of this invention, the antigen, antibody or at least a part of the antibody is immobilized on the liposomes by using a cross-linking agent or an activator for a lipid with the aid of a covalent bond between atoms.

In this invention, an amount of the phospholipid and/or glycolipid constituting the liposomes is preferably such that an amount of the above compound which has reacted with the cross-linking agent is within the range of 0.01 to 30 mol %, in the case a cross-linking method is employed. Further, it is preferred that a concentration of the hydrophilic antigen, antibody or at least a part of the antibody to be immobilized is within the range of 0.01 to 20 mg/ml with respect to 0.5 mM of the liposomes in terms of the lipid.

When the amount is out of above range, a prepared reagent is not applicable for practical use since the sentivity for measurement and stability of the prepared liposomes are greatly affected depending on an amount of the lipid to be reacted with cross-linking agent and a concentration of a protein to be immobilized.

In the reagent of this invention, the antigen, antibody or at least a part of the antibody is immobilized on the liposomes by using an undermentioned cross-linking agent such as N-succinimidyl-3-(2-pyridyldithio)propionate with the aid of a covalent bond between atoms.

The marker material to be enclosed in the liposomes must be hydrophilic and must be determinable when released out of the liposomes. Examples of such marker materials

include fluorescent compounds such as carboxy fluorescein which emits no fluorescence due to self quenching at a high concentration but gives off a very intensive fluorescence at a low concentration ($10^{-3}$ M or less); luminous compounds such as luminol and luciferin which emit light owing to an oxidative reaction outside the liposomes; saccharides such as glucose and sucrose which are decomposed by the function of an oxidative fermentation in order to lead to the consumption of oxygen or the production of hydrogen peroxide; relatively great ionic compounds such as tetrapentyl ammonium; coenzymes such as nicotinamidoadeninedinucleotide (NAD); radical compounds such as methylviologen; absorptive compounds having absorption bands specific to a visible region or ultraviolet region such as water-soluble metallic indicators, e.g. Xylenol Orange (XO), Methylxylenol Blue (MXB), Methylthymol Blue (MTB), water-soluble PADAP (5-Br-PAPS), water-soluble PADAB (5-Br-PSAA), Bismuthiol II, Pyrocatechol Violet (PV) and Eriochrome Black T (BT), and water-soluble dyes, e.g. Rhodamine B, Oxamine Red X and Acid Orange I; and enzymes such as alkaline phosphatase, glucose oxidase, peroxidase, luciferase and the like.

These compounds mentioned above can be suitably selected taking, into consideration, factors such as a detecting manner, a sensitivity and a stability of the liposomes.

The reagent for the immunoassay regarding this invention can react with the antigen or antibody to be detected, which is included in the sample, and the separately added complement in order to discharge a marker material from inside of the liposomes. Then, the discharged maker material is measured by means of a method with respect to the discharged marker material, whereby the quantitative analysis of the antibody or antigen included in the sample can easily be carried out.

The reagent for the immunoassay of this invention described hereinbefore can be prepared, for example, in the following method: First of all, a desired lipid is reacted with a cross-linking agent (a method of using such a cross-linking agent is called a cross-linking method) in a solvent (in place of the cross-linking agent, an activator for the lipid may be used, and a method of using such an activator is called an active lipid method), and functional groups which will be able to combine with the antigen, antibody or at least a part of the antibody immobilized on the liposomes are introduced into the lipid molecules. Next, the thus obtained functional lipid, cholesterol and, if necessary, another lipid are placed in a flask, and a solvent is further added thereto. After mixing, evaporation for the removal of the solvent and suction to dryness are followed. Subsequently, a selected aqueous marker material solution is added to the flask on the inner wall surface of which a thin membrane is formed, and it is sealed with a cap. After stirring, a suspension of the immobilized liposomes is prepared.

On the other hand, the antigen, antibody or at least a part of the antibody to be immobilized on the liposomes is reacted with the cross-linking agent in a buffer solution to introduce cross-linking groups thereinto. After this step, if necessary, the antigen or antibody is further reacted with a reagent (e.g., dithiothreitol (DTT)) for reducing the cross-linking groups, thereby obtaining the modified antigen, antibody or at least a part of the antibody. This process can be omitted, when the lipid is treated with its activator in the preceding process.

In the last place, the immobilized liposomes are reacted with the modified antigen, antibody or at least a part of the antibody (in the case that the active lipid method is

employed, the unmodified antigen, antibody or at least a part of the antibody) in the buffer solution in order to prepare the reagent for the immunoassay of this invention. In general, such a reagent is obtained in the form of liposomes which include the marker material therein and support the immobilized antigen, antibody or at least a part of the antibody on the surfaces thereof. At this time, it is important to suitably vary a concentration of the immobilized modified antigen, antibody or at least a part of the antibody in compliance with a kind of target to be measured.

Examples of the cross-linking agents used in the abovementioned cross-linking method include N-succinimidyl-3-(2-pyridyldithio)propionate (SPDP), N-succinimidyl-4-(p-maleimidophenyl)butyrate (SMPB), N-succinimidyl-4-(p-maleimidophenyl)acetate (SMPA), N-succinimidyl-4-(p-maleimidophenyl)propionate (SMPP), N-(γ-maleimidobutyryloxy)-succinimide (GMBS) and N-(ε-maleimidocaproyloxy)succinimide (EMCS).

The above compound SPDP is represented by the following formula:

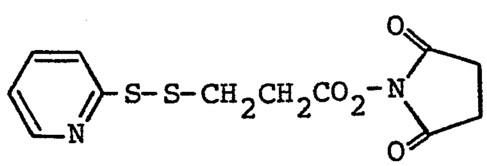

This compound SPDP reacts under moderate conditions to couple compounds having primary amino groups to each other, and it is commercially available from Pharmacia Inc. This cross-linking agent can be utilized, for example, as follows: The protein antigen to be immobilized is treated with the agent SPDP and is reduced with dithiothreitol (DTT). Then, it is reacted with the liposomes which has previously been treated with SPDP. After allowed to stand at room temperature or less for a

period of several hours to one day, the antigen can be immobilized on the liposomes.

The above-mentioned compound SMPB is represented by the following formula:

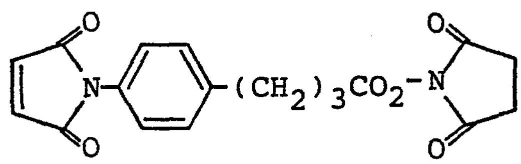

This compound SMPB can immobilize a protein by a reaction similar to that of SPDP. However, in the case of SMPB, an -S-S- bond is not included in the end product (an -S- bond is only included therein). Thus, the end product is stable even under a reducing atmosphere such as in a serum.

On the other hand, examples of the activators for the lipids include cyanogen bromide (CNBr), cyanuric chloride (CC), epichlorohydrin (EH), O-bromoacetyl-N-hydroxysuccinimide and 1,4-bis(2,3-epoxypropoxy)butane (BEPB). Of these compounds, CNBr, CC, EH and BEPB serve to activate compounds having saccharide residues and to couple them to compounds having primary amino groups. Therefore, when the saccharide residues are present on the liposomes, these reagents can be applied. Also in the case that the antigen itself is a glycoprotein and the primary amino groups are present on the liposomes, these activators are likewise effective.

If the cross-linking agents or the activators for the lipids which have been mentioned hereinbefore are employed, the immobilization of the hydrophilic antigen, antibody or at least a part of the antibody on the liposomes will be feasible, though such an immobilization has been impossible in former days. In the method of this invention, there can be avoided such a phenomenon as

the deterioration in the activity of the antigen or antibody at the time of the immobilization with a strong cross-linking agent, e.g., glutaraldehyde (hereinafter referred to as GA).

Moreover, the reagent for the immunoassay of this invention may be manufactured by first coupling the lipid to the antigen, antibody or at least a part of the antibody with the aid of the cross-linking agent or the activator for the lipid; adding the coupled material together with a surface active agent to water in order to form micells; and removing the surface active agent therefrom by means of dialysis or gel filtration.

Materials which can be determined by the above-mentioned reagent for the immunoassay widely range and include antigens of tumor markers (the above-mentioned AFP, BEP, CEA, POA, etc.), immunoglobulins (IgA, IgE, IgG, IgM, etc.), hormones (insulin, $T_3$, $T_4$, etc.), drugs (phenitoin, digoxin, etc.) and the like; and antibodies or at least a part of the antibodies corresponding to the aforesaid antigens. These materials to be detected which will bring about an antigen-antibody reaction with the immobilized antigen, antibody or at least a part of the antibody may not always be hydrophilic.

According to the method for the immunoassay of this invention, the reagent for the immunoassay described above is mixed with a sample including the antigen or antibody (if the antigen is immobilized on the liposomes, the antibody sample will be used; if the antibody or at least a part of the antibody is done, the antigen sample will be used) and a complement in a suitable buffer solution (e.g., a gelatin-Veronal buffer solution [hereinafter referred to as $GVB^-$] including, at ultimate concentrations, 0.1 mM $MgCl_2$ and 0.15 mM $CaCl_2$, respectively) in order to bring about a coupling reaction of

the antigen-antibody with the complement. Then, a marker material is released out of the liposomes in proportion of their reacting amount. Next, determination is carried out by an analytical method (e.g., if the marker material has fluorecsence, a fluorescent analytical method will be emploed) in accordance with this marker material, and an amount of the antigen or antibody in the sample can be measured on the basis of a previously prepared calibration curve.

The complements used in the determining operation are not particularly limited, but the preferred complements each have a high activity, i.e. a high complement value and preferably in the range from 0.1 to 10 $HC_{50}$. Usually, a guinea pig serum is preferably employed. However, sera of rabbit, mouse, human and the like may also be applicable. In the determination of the antigen or antibody by the use of the reagent for the immunoassay of this invention, the complement value is an important factor to decide a measurement range and a detection limit, and better measurement results can be obtained by changing this complement value variously.

Further, if this invention is applied to the preparation of an analytical reagent in which a substrate or enzyme is immobilized on the liposomes, an enzyme or substrate in a sample can also be quantitatively analyzed.

According to the reagent for the immunoassay of this invention, the hydrophilic antigen, antibody or at least a part of the antibody is immobilized on the liposomes without deteriorating its activity, and since the marker material is enclosed in the liposomes, the accurate determination, having a high detection sensitivity, of the antigen or antibody can be carried out in a homogeneous system in a short time. In addition thereto, the analytical method of this invention can be applied to an

extensive region of the materials to be detected and can be accomplished at lower costs. Further, the present invention permits facilitating a highly accurate automatic analysis in miniature and simultaneously measuring many components.

Now, this invention will be descirbed in detail as examples, but these examples never intend to limit the scope of this invention.

Example 1

Measurement (I) of an anti-human immunoglobulin G (IgG) antibody by the use of human IgG immobilized liposomes

(A) Reagents and preparation of the immobilized liposomes

(1) Reagents

There were used dipalmitoylphosphatidyl choline (DPPC), cholesterol, dipalmitoylphosphatidyl ethanolamine (DPPE) and dithiothreitol (DTT) which were commercially available from Sigma Inc. N-succinimidyl-3-(2-pyridyldithio)-propionate (SPDP) and Sephadex G-25 Fine were bought from Pharmasia Fine Chemicals Inc. Other reagents were commercially available ones (special grade), which were directly used without any purification. Further, ionexchange water was used as water.

(2) Preparation of the immobilized liposomes

a) Preparation of DPPE-dithiopyridinate (DPPE-DTP)

In a test tube were placed 5 ml of 10 mM DPPE (a chloroform solution) and 50 mg of SPDP, and after replaced with a nitrogen gas and sealing, reaction was carried out at

room temperature for 2 hours. After the reaction, the reaction product was extracted three times with physiological saline in a five-fold amount, and the remaining chloroform phase was subjected to a vacuum drying treatment. At the last step, 5 ml of chloroform were added thereto, and the product was then stored in a sealed test tube at - 20 °C.

b) Preparation of the liposomes

All of a used lipid was dissolved in chloroform or a chloroform/methanol solution (2/1). In the first place, 200 µl of 5 mM DPPC, 100 µl of 10 mM cholesterol and 60 µl of 1 mM DPPE-DTP were placed in a 10 ml eggplant type flask and 2 ml of chloroform were further added thereto, followed by sufficient stirring. The used solvent was removed therefrom in a water bath (about 50 °C) by means of a rotary evaporator. Once again, 2 ml of chloroform were added thereto, and after enough stirring, the solvent was evaporated away by the rotary evaporator again. When this operation was repeated several times, a thin membrane was formed on the wall surface of the flask. The flask was then transferred into a desicator and suction was carried out for about one hour by a vacuum pump in order to absolutely remove the solvent. Next, 100 µl of 0.2 M carboxyfluorescein (Eastman Kodak Inc.; pH 7.4) were added thereto, and the interior of the flask was replaced with nitrogen. After sealing, the flask was immersed in a water bath at 60°C or so for about one minute. Continuously, vigorous stirring was carried out in the flask by a Vortex mixer till the thin lipid membrane on the wall surface thereof completely disappeared. By virtue of this operation, a liposomes suspension was prepared. A small amount of a buffer solution ($GVB^-$ including, at ultimate concentrations, 0.1 mM $MgCl_2$ and 0.15 mM $CaCl_2$, respectively; hereinafter merely referred to as $GVB^{2+}$) was added thereto, and all

of the liposomes suspension was transferred to a centrifuge tube. Centrifugal treatment was carried out at 4 °C and 15,000 rpm for 20 minutes in order to remove free carboxyfluorescein. This treatment was repeated using $GVB^{2+}$ till the supernatant became transparent. In the last place, 2 ml of $GVB^{2+}$ and 5 µl of 10 % $NaN_3$ were added thereto and were suspended by means of the Vortex mixer. After introducing nitrogen and sealing, the product was stored in a refrigerator.

c) Modification of human IgG

In 2 ml of a 0.01 M HEPES buffer solution (including 0.85 % of NaCl; pH 7.45) were dissolved 5 mg of human IgG (available from Miles Inc.). After replaced with nitrogen, 10 µl of 10 mM SPDP (an ethanol solution) were added thereto. The resulting mixture was sufficiently stirred and was reacted for 30 minutes in situ. After the reaction, the reaction mixture was developed through a column (gel volume : about 15 ml) filled with Sephadex G-25 Fine gel which was previously saturated with a physiological saline, and elution was carried out with a 0.1 M acetic acid buffer solution (including 0.85 % of NaCl; pH 4.5). To a first peak fraction (about 2 ml) were further added 2 ml of the acetic acid buffer solution, and after replaced with nitrogen, about 30 mg of dithiothretol were added thereto. Reaction was carried out over enough stirring for 20 minutes. After the reaction, the reaction mixture was developed through a column (gel volume : about 30 ml) filled with Sephadex G-25 Fine gel which was previously saturated with a 0.01 M HEPES buffer solution, and elution was carried out with the HEPES buffer solution. A first peak fraction (about 2 ml) was collected, and after nitrogen replacement, the product was stored in a refrigerator till used.

d) Preparation of human IgG immobilized liposomes

The liposomes suspension prepared above was mixed with an equal amount of the modified human IgG solution, and after nitrogen replacement and sealing, reaction was carried out overnight at room temperature with slowly shaking, followed by washing with the HEPES buffer solution and next with $GVB^{2+}$ in order to remove unreacted human IgG. $GVB^{2+}$ in an amount equal to that of the liposomes suspesion used in the reaction, and 5 µl of 10 % $NaN_3$ were lastly added, and after suspending and nitrogen replacement, the product was stored in a refrigerator till used.

(3) Measurement of an anti-human IgG antibody by the use of the IgG immobilized liposomes

Into each of wells on a U-shaped plate (96 holes) made by Nunk Inc. were poured 25 µl of an anti-human IgG antibody which was diluted with a suitable amount of $GVB^{2+}$ 100-fold, and was poured into each well in an amount of 5 µl. In the last place, a complement (guinea pig serum) suitably diluted with $GVB^{2+}$ was added every 25 µl. Reaction was carried out at a constant temperature of 37 $^{o}$C for 1.5 hours. After the reaction, 100 µl of a 0.01 M EDTA-Veronal buffer solution were added to each well in order to stop the reaction, and a fluorescence of each well was measured by means of a fluorospectrophotometer for a plate (MFP-12F, available from Corona Electronic Co., Ltd.; Ex = 490 nm, Em = 520 nm). Measured values were represented with relative values by regarding, as 100 %, a difference between a fluorescence of the well in which 25 µl of 10 % Triton X-100 (available from Rohm & Haas Co.) were substituted for the antibody and that of the well in which 25 µl of $GVB^{2+}$ were substituted for the antibody. The results obtained by employing the the 400-fold diluted complement (line A) and the 800-fold

diluted complement (line B) are shown in Figure 1, respectively.

Example 2

<u>Measurement (II) of an anti-human IgG antibody by the use of human IgG immobilized liposomes</u>

(1) Preparation of the immobilized liposomes

Following the same procedure as in Example 1, the liposomes including carboxyfluorescein was prepared. After centrifugal washing, the liposomes were suspended in a 0.1 M phosphoric acid buffer solution (including 0.85 % of NaCl; pH 6.5) (2 ml), and 150 mg of DTT were then added thereto. After nitorgen replacement, stirring was sufficiently carried out and reaction was done at room temperature for 2 hours in situ. Washing was carried out (by using $GNV^{2+}$ already replaced with nitrogen) centrifugally (15,000 rpm, 20 minutes) and 5 µl of 10 % $NaN_3$ were added thereto, and the resulting product was stored in a refrigerator. On the other hand, an antigen was treated with SPDP in the same manner as in Example 1 and was separated by a gel filtration (elution with an HEPES buffer solution) and purified. The prepared liposomes suspension was mixed with the modified human IgG solution in equal amounts, and after nitrogen replacement, reaction was carried out overnight at room temperature with slowly shaking. After the reaction, washing was sufficiently done (by using $GVB^{2+}$ already replaced with nitrogen), and 5 µl of 10 % $NaN_3$ were then added thereto. Afterward, the product was stored in a refrigerator. Incidentally, the liposomes which will be applied to the coupling reaction with modified human IgG is preferably treated with DTT just before the coupling step, but in the case that the liposomes coolingly stored is unavoidably used, it is preferred that a small amount (10 to 20

mg) of DTT is added thereto again before the coupling reaction and the reaction is then carried out at room temperature for 30 minutes or so. This process serves to cut off S-S bonds formed between the liposomes during the storage.

(2) Measurement of an anti-human IgG antibody by the use of the human IgG immobilized liposomes

Following the same procedure as in Example 1, an anti-human IgG antibody was measured using the liposomes, and results similar to those of Example 1 were obtained.

Example 3

Measurement of human IgG in a human serum by the use of the human IgG immobilized liposomes

The liposomes, prepared in Example 1, on which human IgG was immobilized was utilized to measure an amount of human IgG in the human serum. Into each line on a microtiter plate, a 1,000-fold, a 10,000-fold and a 20,000-fold diluted (by using $GVB^{2+}$) anti-human IgG antibody were poured every 25 µl, and samples of human sera which were diluted 10 to $10^6$ times were added thereto every 25 µl. Reaction was then carried out overnight at 4 °C. Next, the 100-fold diluted liposomes on which human IgG was immobilized was added thereto every 5 µl and a 400-fold or a 800-fold diluted complement (guinea pig serum) was lastly added thereto every 25 µl, and the resulting mixture was allowed to stand at 37 °C for 1.5 hours. Subsequent procedures were the same as described in the preceding paragraph regarding the measurement of the anti-human IgG antibody in Example 1. The experimental results are shown in Figure 2. On the other hand, a solution including human IgG of which concentration was known was utilized and similar

procedures were repeated in order to prepare a calibration curve with respect to the concentration of human IgG. An amount of human IgG in the human serum could be determined by utilizing this calibration curve.

Example 4

<u>Measurement of human AFP by the use of a human AFP immobilized liposomes</u>

Following the method described in Example 1, human AFP (commercially available from Nippon Biotest Laboratory) was immobilized on the surface of liposomes including carboxyfluorescein. On the other hand, in the same manner as in Example 3, a suitably diluted (100 to 1000 ng/ml) (by using $GVB^{2+}$) anti-human AFP antibody solution was poured every 25 µl into wells on a microtiter plate, and a human AFP standard solution (3 to 1000 ng/ml; available from Nippon Biotest Laboratory) were added every 5 µl to each pair of two wells. Then, reaction was carried out at room temperature (about 25 °C) for 30 minutes. Afterward, 5 µl of the above-mentioned human AFP immobilized liposomes (which was diluted 100 times with $GVB^{2+}$) and guinea pig serum (which was diluted 400 times with with $GVB^{2+}$) were added thereto, and the resulting mixture was allowed to sand at 37 °C for 1.5 hours. After that, 100 µl of an EDTA solution were added thereto to stop the reaction. Fluorospectrophotometer was employed for the determination of fluorescence in each well and the measured values were plotted with respect to a concentration of human AFP. For example, when the 100 ng/ml anti-human AFP could be quantitatively analyzed within the range of 10 to 500 ng/ml. A concentration of AFP in an unknown sample could be determined by using a calibration curve within such a range.

Example 5

Immobilization of human IgG on the liposomes including CNBr-activated hematoside (glycolipid)

(1) Preparation of the CNBr-activated hematoside

In 30 ml of chloroform/methanol (2 : 1) were dissolved 50 mg of hematoside ($GM_3$), and 5N NaOH was dropwise added thereto in order to adjust a pH of the solution to 10.5. While the solution was stirred by a stirrer, 1 g of CNBr (which was dissolved in several milliliters of methanol) was added thereto, and 5 N NaOH was immediately added thereto in a dropwise manner in order to adjust its pH into a range of 10 to 11. After several minutes, the reaction mixture was transferred to a separatory funnel and 5 ml of distilled water were added thereto, followed by vigorous stirring. After 10 minutes, an lower layer therein was taken out to obtain CNBr-activated hematoside.

(2) Immobilization of human IgG on the liposomes

The liposomes were prepared in the same manner as in Example 1, and 5 mg of human IgG were added to this liposomes suspension. Reaction was carried out at room temperature for 3 hours. After washing with $GVB^-$, the liposomes were suspended in 2 ml of $GVB^-$ again and were then preserved in a refrigerator after the introduction of nitrogen.

(3) Quantitative analysis of anti-human IgG antibody (rabbit)

As a result of the quantitative analysis of the antibody in all the same procedure as in Example 1, it was definite that the determination of the antibody is

feasible within the range of $10^{-8}$ to $10^{-10}$ g/ml.

Example 6

Measurement (I) of an anti-human IgG antibody by the use of human IgG immobilized liposomes

(A) Reagents and preparation of the liposomes

(1) Reagents

The used reagents were the same as in Example 1.

(2) Preparation of the immobilized liposomes

a) Preparation of DPPE-dithiopyridinate (DPPE-DTP)

In a conical flask equipped with a stopper were placed 70 g of DPPE, and the latter was dissolved in 25 ml of a chloroform/methanol (5 : 1) solution. After the addition of 60 ml of triethanolamine and 50 mg of SPDP, nitrogen replacement was carried out. Reaction was carried out at room temperature for one hour and the solvent was then removed from the system by means of a rotary evaporator. The resulting dried material was dissolved in 5 ml of a chloroform/methanol (10 : 1) solution and was purified with a silica gel column chromatography. The resulting product fractions were collected and were concentrated to about 5 ml by an evaporator. The yield was within the range of 80 to 95%. The storage was done at -20 °C under the introduced nitrogen gas.

b) Preparation of the liposomes

The procedure of the liposomes preparation in Example 1 was repeated with the exception that an amount of DPPE-DTP was 50 µl and an equal amount of 0.2 M water-soluble PADAP (5-Br-PAPS) was substituted for carboxy fluorescein.

c) Modification of human IgG

The procedure of the Example 1 was repeated with the exception that 5 mg of human IgG (available from Miles-Yeda Inc.) were used, and elution was carried out with the HEPES buffer solution. An initial protein fraction (about 2 ml) was collected, and after nitrogen replacement, it was stored in a refrigerator till used.

d) Preparation of human IgG immobilized liposomes

The same procedure in Example 1 was repeated in order to prepare the liposomes on which human IgG was immobilized.

(3) Measurement of an anti-human IgG antibody by the use of the human IgG immobilized liposomes

$GVB^{2+}$ ($GVB^{-}$ including, at ultimate concentrations, 0.1 mM $MgCl_2$ and 0.15 mM $CaCl_2$, respectively) having pH 7.8 was poured into cells every 25 µl. Next, the above-mentioned liposomes suspension was diluted with $GVB^{2+}$ 100-fold and was poured into each cell every 5 µl.

In the last place, a complement (guinea pig serum) suitably diluted with $GVB^{2+}$ was added thereto every 25 µl, and reaction was carried out at 37 °C for an hour. Measurement of the optical density was made at an absorption wavelength of 550 nm.

The measured values were represented with relative values by regarding, as 100 %, a difference between an absorption in the case that Triton X-100 and $GVB^{2+}$ were used in an amount of 25 µl, respectively, in place of the antibody and complement and an absorption in the case

that 25 µl of $GVB^{2+}$ were used in place of the antibody. The results obtained by using the 400-fold diluted complement (complement value = 0.5 $CH_{50}$) are shown in Figure 3. This Figure 3 indicates that the anti-human IgG antibody can be determined within the range of $10^{-3}$ to $10^{-5}$ mg/ml.

Example 7

In all the same manner as in Example 1, liposomes on which human IgG was immobilized was prepared.

The measurement of an anti-human IgG antibody was made using these human IgG immobilized liposomes, as follows:

Into a U-shaped plate (96 holes) made by Nunk Inc. were poured 25 µl of an anti-human IgG antibody which was diluted with a suitable amount of $GVB^{2+}$. Next, the liposomes suspension was diluted with $GVB^{2+}$ 100-fold, and was poured into each well in an amount of 5 µl. In the last place, a complement (guinea pig serum) suitably diluted with $GVB^{2+}$ was added thereto every 25 µl. Reaction was carried out at a constant temperature of 37 $^{o}$C for 1.5 hours. After the reaction, 100 µl of a 0.01 M EDTA- Veronal buffer solution were added to each well in order to stop the reaction, and a fluorescence of each well was measured by means of a fluorospectrophotometer (MFP-12F, Corona Electronic Co., Ltd.) for the plate (Ex = 490 nm, Em = 520 nm). Measured values were represented with relative values by regarding, as 100 %, a difference between a fluorescence of the well in which of 10 % Triton X-100 and $GVB^{2+}$ were substituted in an amount of 25 µl respectively for the antibody and complement, and that of the well in which 25 µl of $GVB^{2+}$ were substituted for the antibody. The results obtained by employing the the 400-fold diluted complement (complement value = 0.5 $CH_{50}$) are shown in Figure 4.

The curve a in Figure 4 shows a relation between a concentration of the anti-human IgG antibody included in the sample and a relative release rate of the marker material which was released by the reaction of the reagent for the immunoassay of this invention. As be apparent from the drawing, a definite relative relation as present between both of these factors, and thus the quantitative analysis of the sample is feasible on the basis of this fact. On the other hand, the curve b in the drawing exhibits a relation therebetween in the case that glutaraldehyde (GA) disclosed in conventional techniques was used as the cross-linking agent and the liposomes comprising DPPC, cholesterol and DPPE-DTP in a molar ratio of 1 : 1 : 0.05 were employed. As the curve b indicates, the liposomes in which the conventional cross-linking agent was used scarcely change the release proportion of the marker material in accordance with the variation of the sample concentration. By this fact, it has been confirmed that such liposomes cannot be applied to an accurate quantitative analysis.

Example 8

Following the procedure of Example 7, human IgG immobilized liposomes were prepared by changing a content of DPPE-DTP in the liposomes, in other words, by varying an amount of the lipid reacted with a cross-linking agent. The immobilized liposomes were measured for a release rate of CF from the immobilized liposomes when 2 x $10^{-3}$ mg/ml of the anti-human IgG antibody and the 400-fold diluted complement (complement value = 0.5 $CH_{50}$) were acted thereon. The results are shown in Figure 5. As be apparent from this drawing, the release of CF was not observed at all in the case of the liposomes including no DPPE-DTP. In practice, a usable range was from 0.01 mol, and when the content of DPPE-DTP was increased, the CF release rate increased up to 1 mol %. However, when it

was added in excess of this level, the CF release rate did not vary. Further, in the case of the liposomes including 30 mol % or more of DPPE-DTP, the spontaneous CF release was noticeably observed, and the stability was poor. By the results of this example, it was confirmed that constitutional ratios of the phospholipid and glycolipid reacted with the cross-linking agent are within the range of 0.01 to 30 mol %.

Example 9

In Example 7, a concentration of human IgG was variously changed when it was immobilized on the liposomes comprising DPPC, cholesterol and DPPE-DTP in a molar ratio of 1 : 1 : 0.05, with the intention of inspecting a reactivity of the anti-human IgG antibody with the liposomes.

Concentrations of the antibody and complement were the same as in Example 8. The results are shown in Figure 6. The liposomes on which no human IgG was immobilized did not react with the antibody at all. On the contrary, in the case of the liposomes on which the antigen was immobilized, an effective reaction to the antibody concentration of 0.01 mg/ml with respect to 0.5 mM of the liposomes in terms of an amount of the lipid used therein, to 20 mg/ml via a maximum point.

Example 10

A reactivity between the human IgG immobilized liposomes prepared in Example 7 and 2 x $10^{-3}$ mg/ml of the anti-human IgG antibody was inspected by employing various complement values. As shown in Figure 7, when the complement values were 0.1 $CH_{50}$, CF was released from almost all the liposomes even under conditions including no antibody. Therefore, it was impossible to detect the antibody.

As understood from the foregoing, by selecting the concentration of the cross-linking agent to be introduced into the liposomes, the concentration of the antigen and the complement value so that they may be within the range described in Example 7 to 10, a measurement system can be constituted in compliance with a material to be detected and its concentration.

Example 11

In the same manner as in Example 7, the liposomes on which an anti-human IgG antibody was immobilized was prepared. By the use of these liposomes, the determination of human IgG was carried out. In this case, a complement value was 2 $CH_{50}$. The results are shown in Figure 8. It was confirmed that the determination of the antigen could be made also by using the antibody-immobilized liposomes, similarly to the employment of the antigen-immobilized liposomes.

Further, also by using liposomes on which an anti-human CEA antibody, an anti-human AFP antibody and the like are immobilized, antigens could be determined in a similar manner.

Example 12

With regard to the marker (contained) material in the human IgG immobilized liposomes in Example 1, investigation was given as follows:

In the first place, glucose oxidase (a 5 % solution) which was an enzyme was introduced into the liposomes. To an antibody solution which was a sample, glucose the last concentration of which was 500 µM was added, and the procedure of Example 1 was repeated. After 30 minutes' reaction at 37 °C, the decrease in dissolved oxygen was

measured by an oxygen electrode. Figure 9 shows the relation between a dilution rate of the antibody and a relative release rate which was obtained by regarding, as 100 %, an release rate in the case that 10 % Triton X-100 was added in place of the antibody.

Next, a luciferin (0.1 M) solution was introduced into the liposomes, and ATP having a last concentration of 1 mM and a 0.1 % luciferase solution were added to an antibody solution which was a sample. After an antigen-antibody reaction, an emission volume within 30 minutes was measured. Figure 10 shows the relation between a relative release rate and an antibody dilution rate, which relative release rate was obtained by regarding, as 100 %, a value obtained in the case that 10 % Triton X-100 was used.

From the above-mentioned results, it was confirmed that also when enzyme or the luminescent material was introduced into the liposomes, there could be obtained determination results which were substantially similar to those of the fluorescent material.

Claims:

1. A reagent for an immunoassay which comprises liposomes; a hydrophilic antigen, antibody or at least a part of the antibody immobilized on said liposomes by a cross-linking method or an active lipid method; and a hydrophilic marker material enclosed in the liposomes.

2. The reagent for an immunoassay according to Claims 1, wherein a cross-linking agent used in said cross-linking agent method is selected from the group consisting of N-succinimidyl-3-(2-pyridyldithio)propionate; N-succinimidyl-4-(p-maleimidophenyl)butylate; N-succinimidyl-4-(p-maleimidophenyl)acetate; N-succinimidyl-4-(p-maleimidophenyl)propionate; N-(γ-maleimidobutyryloxy)succinimide; and N-(ε-maleimidocaproyloxy)succinimide.

3. The reagent for an immunoassay according to Claim 1, wherein an activator for a lipid used in said active lipid method is bromonitrile, bromoacetylhydroxysuccinimide or cyanuric chloride.

4. The reagent for an immunoassay according to Claim 1, wherein said marker material is a fluorescent compound, liminous compound, absorptive compound, saccharide, ionic compound, enzyme, coenzyme or radical compound.

5. The reagent for an immunoassay according to Claim 1, wherein said hydrophilic antigen or antibody is selected from the group consisting of tumor markers of AFP, BFP, CEA or POA; immunoglobulins of IgA, IgE, IgG or IgM; hormones of insulin, $T_3$ or $T_4$; and drugs of phenitoin or digoxin.

6. The reagent for an immunoassay according to Claim 1, wherein said liposomes are composed of a phospholipid and/or glycopilid and cholesterol.

7. The reagent for an immunoassay according to Claim 6, wherein the molar ratio of said cholesterol to said phospholipid and/or glycolipid is between 0.1 and 5.0.

8. The reagent for an immunoassay according to Claim 7, wherein the molar ratio of said cholesterol to said phospholipid and/or glycolipid is 1.

9. The reagent for an immunoassay according to Claim 6, wherein with regard to said phospholipid and/or glycolipid constituting said liposomes, an amount of said phospholipid and/or glycolipid which has reacted with a cross-linking agent in said cross-linking method is within the range of 0.01 to 30 mol %.

10. The reagent for an immunoassay according to Claim 6, wherein a concentration of the hydrophilic antigen, antibody or at least a part of said antibody to be immobilized is within the range of 0.01 to 20 mg/ml with respect to 0.5 mM of said liposomes in terms of the lipid.

11. A method for an immunoassay which comprises the steps of:

mixing, with a sample including an antigen or antibody and a complement, a reagent for the immunoassay comprising liposomes; a hydrophilic antigen, antibody or at least a part of the antibody immobilized on said liposomes by a cross-linking method or an active lipid method; and a hydrophilic marker material enclosed in said liposomes; and

measuring said marker material released out of said liposomes in order to quantitatively determine said antigen or antibody in said sample.

12. The method for an immunoassay according to Claim 11, wherein a complement value of said complement is 0.1 to 10 $CH_{50}$.

FIG. 1

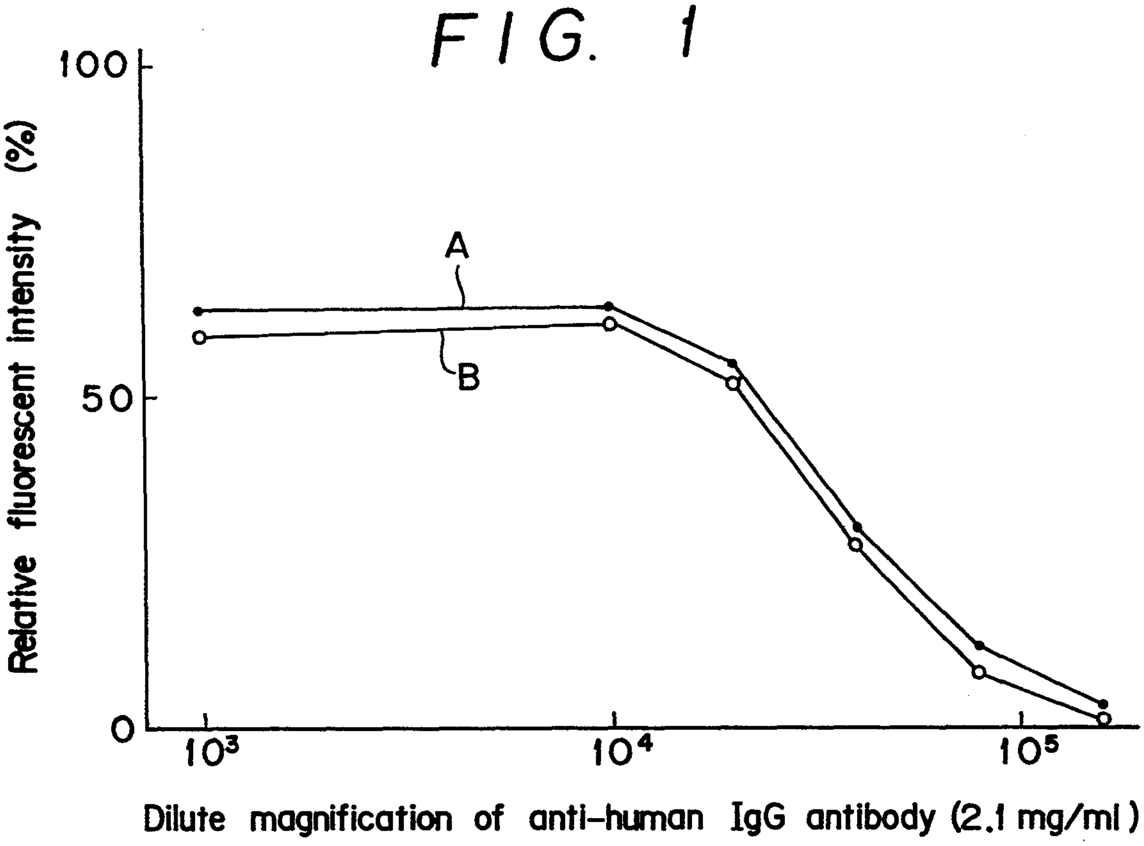
100
50
0
Relative fluorescent intensity (%)
A
B
$10^3$
$10^4$
$10^5$
Dilute magnification of anti-human IgG antibody (2.1 mg/ml)

FIG. 2

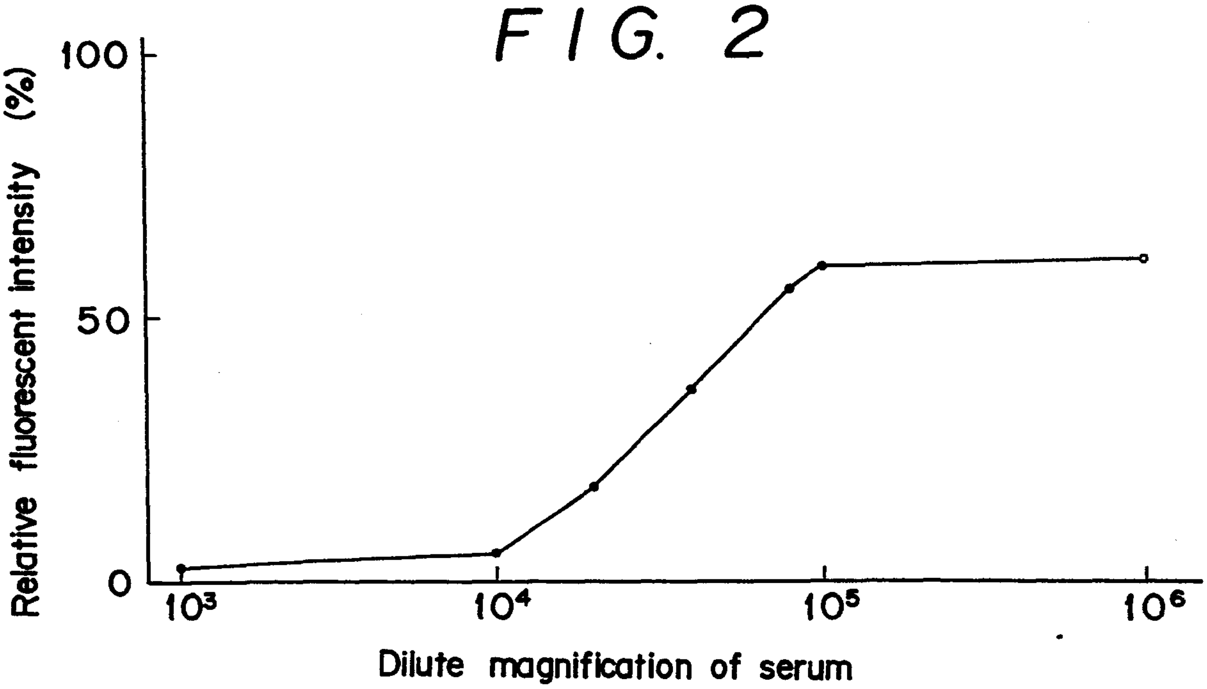
100
50
0
Relative fluorescent intensity (%)
$10^3$
$10^4$
$10^5$
$10^6$
Dilute magnification of serum

FIG. 3

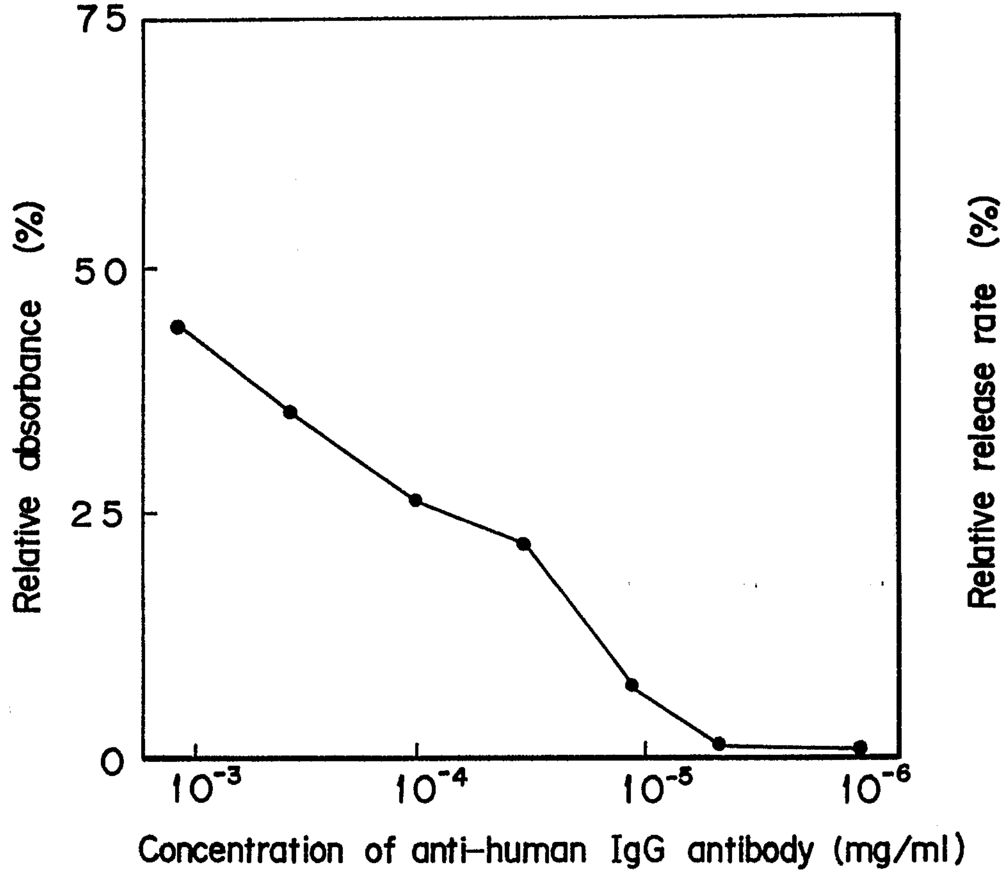
75
50
25
0
Relative absorbance (%)
$10^{-3}$
$10^{-4}$
$10^{-5}$
$10^{-6}$
Concentration of anti-human IgG antibody (mg/ml)

FIG. 4

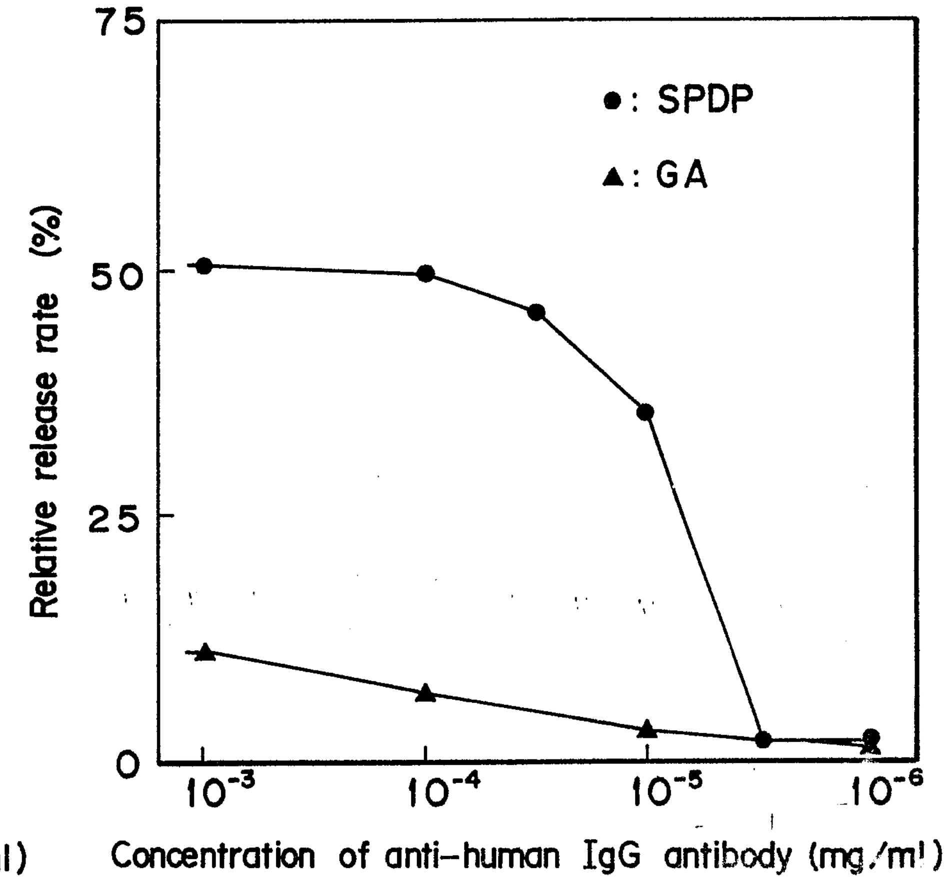
75
50
25
0
Relative release rate (%)
●: SPDP
▲: GA
$10^{-3}$
$10^{-4}$
$10^{-5}$
$10^{-6}$
Concentration of anti-human IgG antibody (mg/ml)

FIG. 5

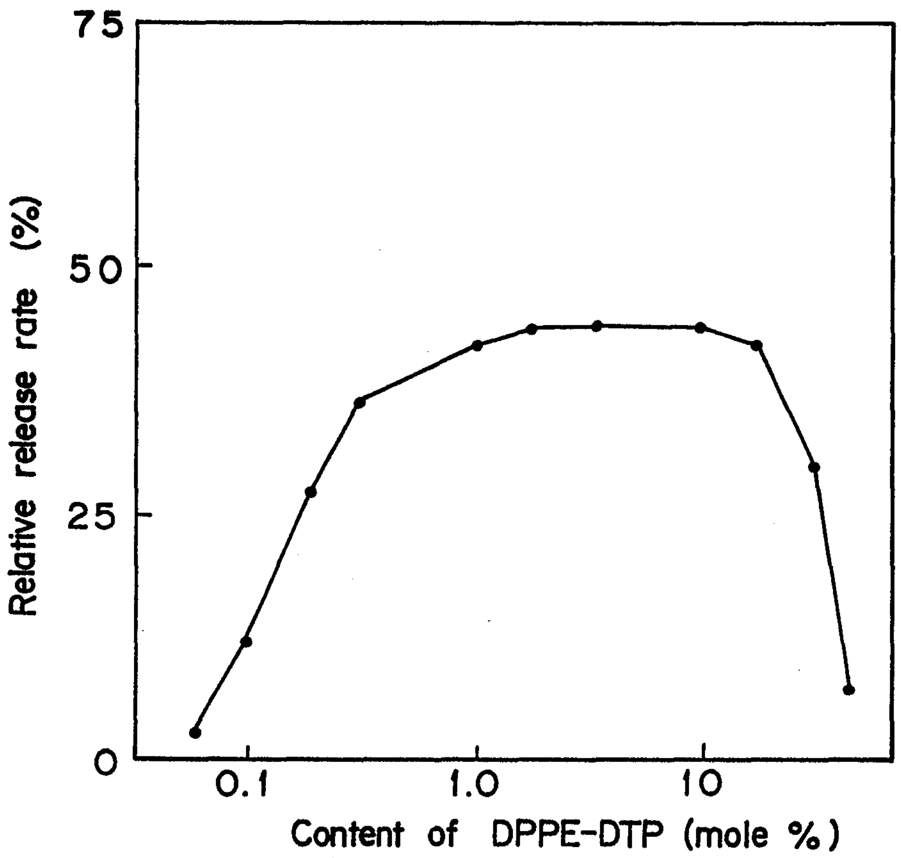
75
50
25
0
Relative release rate (%)
0.1
1.0
10
Content of DPPE-DTP (mole %)

FIG. 6

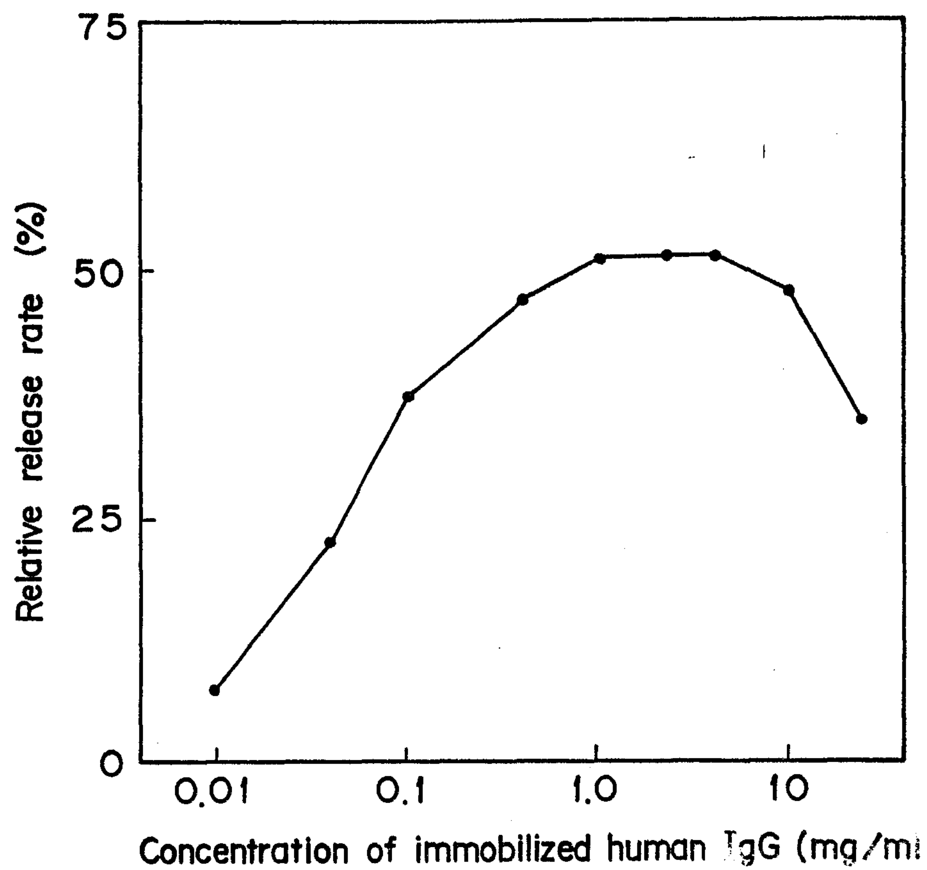
75
50
25
0
Relative release rate (%)
0.01
0.1
1.0
10
Concentration of immobilized human IgG (mg/ml)

FIG. 8

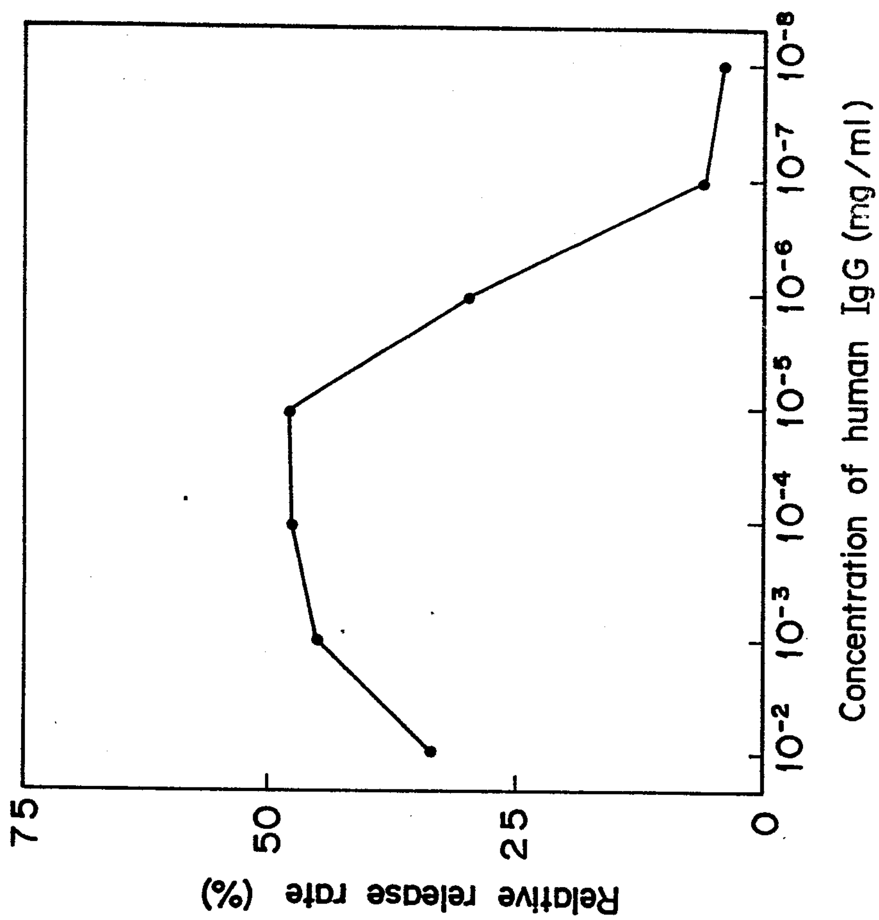
Relative release rate (%)
75
50
25
0
10⁻²
10⁻³
10⁻⁴
10⁻⁵
10⁻⁶
10⁻⁷
10⁻⁸
Concentration of human IgG (mg/ml)

FIG. 7

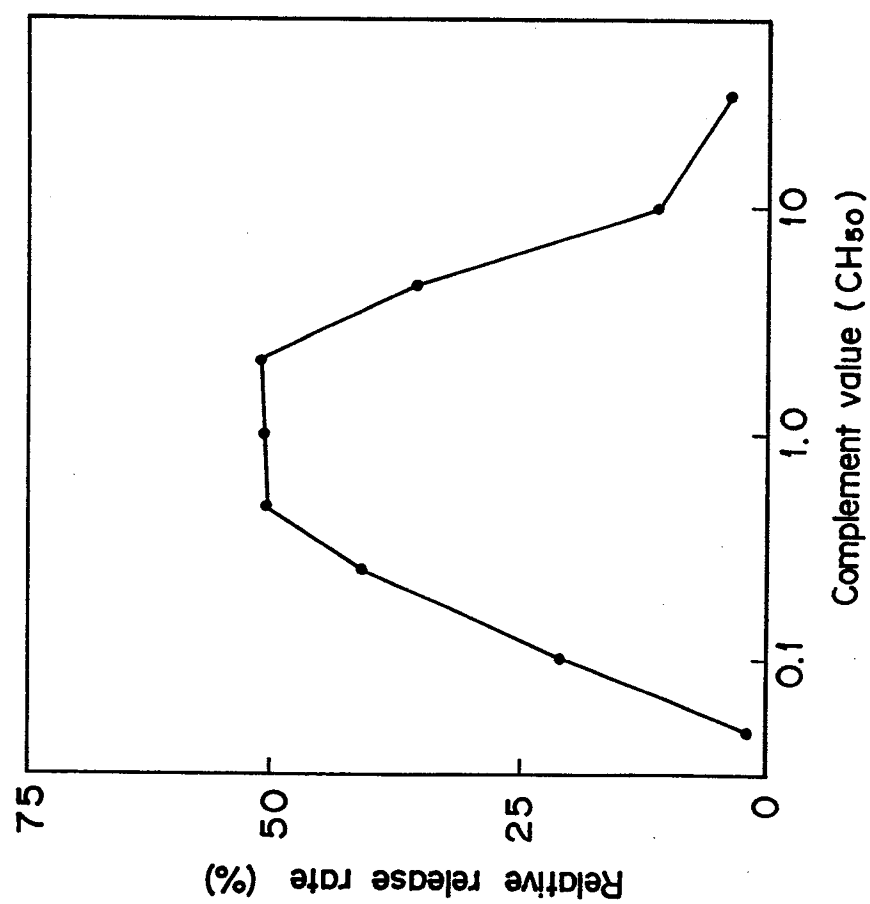
Relative release rate (%)
75
50
25
0
0.1
1.0
10
Complement value (CH50)

FIG. 9

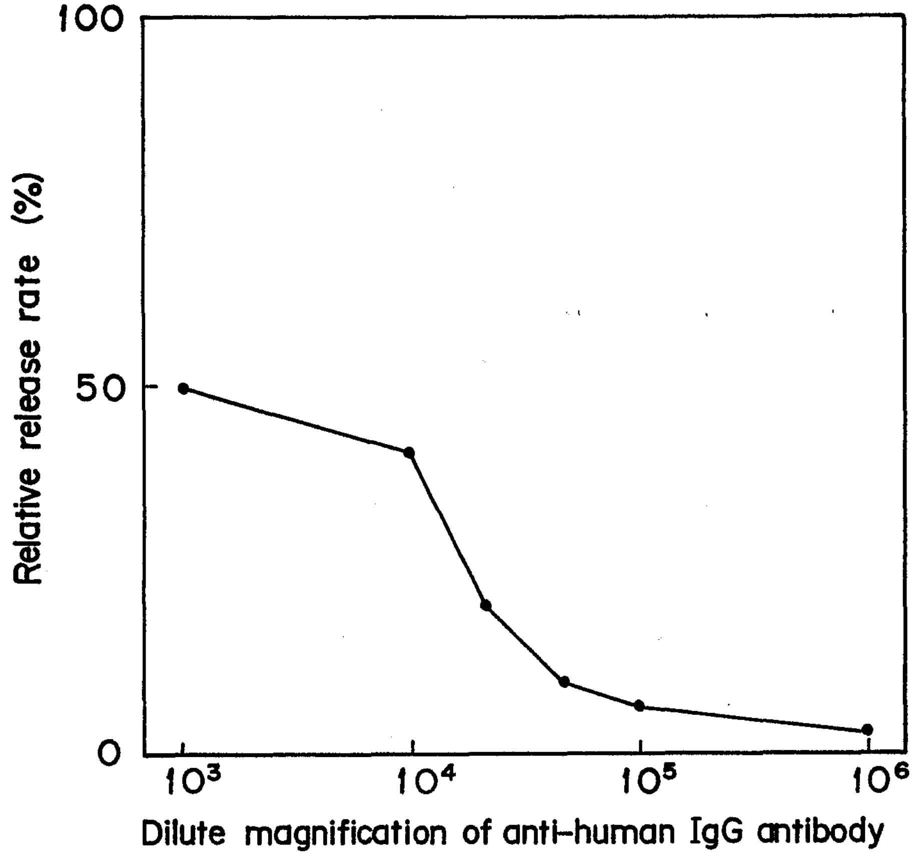
100
50
0
Relative release rate (%)
$10^3$
$10^4$
$10^5$
$10^6$
Dilute magnification of anti-human IgG antibody

FIG. 10

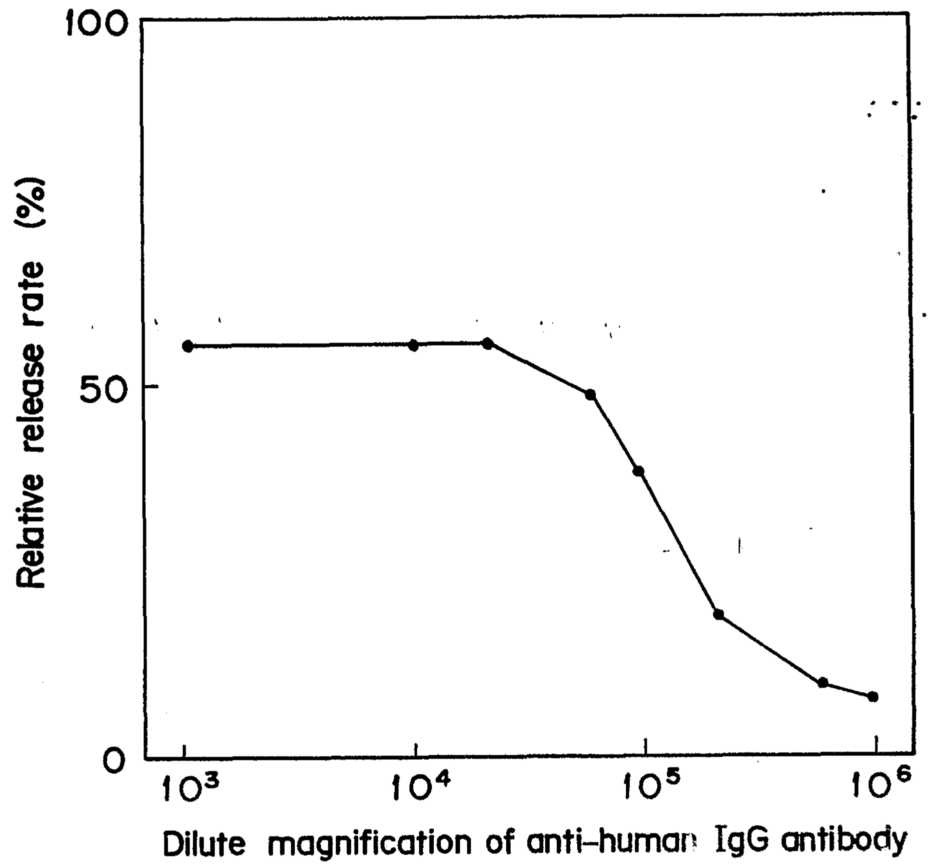
100
50
0
Relative release rate (%)
$10^3$
$10^4$
$10^5$
$10^6$
Dilute magnification of anti-human IgG antibody